# EUROPÄISCHE PATENTSCHRIFT

(11) **EP 3 222 327 B1**
(45) Veröffentlichungstag und Bekanntmachung des Hinweises auf die Patenterteilung: **26.06.2019**
(21) Anmeldenummer: 16162200.6
(22) Anmeldetag: 24.03.2016
(51) Int. Cl.: A61Q 17/00, A61Q 19/00, A61K 8/04, A61K 8/36, A61K 8/362, A61K 8/67, A61K 8/81, A61K 8/86, A61K 8/87, A61K 8/897, A61K 8/19, A61K 8/365

(54) **WÄSSRIGE LÖSUNG AUF DER BASIS VON FLUORPOLYMEREN**
AQUEOUS SOLUTION BASED ON FLUOROPOLYMERS
SOLUTION AQUEUSE A BASE DE FLUOROPOLYMERES

(43) Veröffentlichungstag der Anmeldung: 27.09.2017
(73) Patentinhaber: Herwe GmbH, 74889 Sinsheim-Dühren (DE)
(72) Erfinder: MUNZ, Olaf, 70825 Korntal (DE); HERSCHLEIN, Dominik, 68723 Schwetzingen (DE)
(74) Vertreter: Ellwanger & Baier Patentanwälte Partnerschaftsgesellschaft

(56) Entgegenhaltungen:
- EP-A1- 0 864 317
- EP-A2- 1 074 243
- DATABASE GNPD [Online] MINTEL; Juli 2011 (2011-07), Giuliani: "Medium Protection Cream SPF20", XP002758280, Database accession no. 1592308
- Herwe: "Technisches Merkblatt: Herwesan oll-in-one", , Juli 2015 (2015-07), XP002758282, Gefunden im Internet: URL:http://www.herwe.de/produkt/herwesan-a ll-in-one/ [gefunden am 2016-05-30]
- Herwe: "Herwederm protect sensitive", , 7. Februar 2001 (2001-02-07), XP002758283, Gefunden im Internet: URL:http://www.herwe.de/wp-content/uploads /2015/09/HERWEDERM_PROTECT_SENSITIVE_TMB.p df [gefunden am 2016-05-30]
- DATABASE GNPD MINTEL; Februar 2012 (2012-02), Uriage: "Tech. Gel", XP002758281, Database accession no. 1735151
- Orochemie: "C60 Skin Protection Foam: Product Information", , Mai 2013 (2013-05), XP002758284, Gefunden im Internet: URL:https://www.orochemie.de/en/kurzinfo_o rochemie_c60.php [gefunden am 2016-06-06]

## Beschreibung

Die vorliegenden Erfindung betrifft eine wässrige Lösung (wL), die Wasser, mindestens ein Tensid, mindestens ein Fluorpolymer, mindestens einen basischen pH-Regulator und mindestens einen sauren pH-Regulator enthält, sowie ein Verfahren zu deren Herstellung. Darüber hinaus betrifft die vorliegende Erfindung einen wässrigen Schaum (wS), der die wässrige Lösung (wL) sowie ein Gas enthält, und ein Verfahren zu dessen Herstellung. Außerdem betrifft die vorliegende Erfindung eine Vorrichtung, die die wässrige Lösung (wL) und mindestens ein Gas umfasst und die Verwendung der wässrigen Lösung (wL) und des wässrigen Schaums (wS) als Hautschutzpräparat.

Insbesondere bei Arbeiten mit hautschädigenden Arbeitsstoffen und unter Arbeitsbedingungen, die die Haut schädigen können, besteht Bedarf an Hautschutzprodukten, die die schädigende Wirkung von sowohl wasserlöslichen als auch wasserunlöslichen Arbeitsstoffen auf der Haut minimieren. Im Stand der Technik sind bereits verschiedene derartige Hautschutzprodukte beschrieben. Den jeweiligen Produktdatenblättern oder sonstigen Informationsbroschüren der jeweiligen Hersteller kann folgendes entnommen werden:
"Herwesan all-in-one" und "Herwederm Protect Sensitive" der Firma Herwe werden als Hautschutzpräparate gegen wasserlösliche und wasserunlösliche Arbeitsstoffe eingesetzt (Technisches Merkblatt "Herwesan all-in-one", Revision 5 - Juli 2015 und Technisches Merkblatt "Herwederm Protect Sensitive", Revision 5 - Juli 2015). Diese Hautschutzpräparate zeigen bereits gute Wirkung gegen wasserlösliche und wasserunlösliche Arbeitsstoffe und sind zudem abdruckarm. Diese Hautschutzpräparate sind keine wässrigen tensidischen Lösungen, sondern liegen als Emulsion oder Gel (halbfestes Erzeugnis) vor. Die Verteilbarkeit und Dosiergenauigkeit ist daher limitiert. Es ist möglich, dass sie unregelmäßig verteilt (punktuell unterdosiert) oder überdosiert und dadurch vom Anwender nicht akzeptiert werden.

Um die Wahrscheinlichkeit einer schlechten Verteilung oder einer Überdosierung zu minimieren, sind im Stand der Technik verschiedene Schäume beschrieben, die ebenfalls für den Hautschutz eingesetzt werden können.

Derartige Schäume werden beispielsweise unter dem Handelsnamen "Marly-Skin Hautschutzschaum" der Firma Marly Products und unter dem Handelsnamen "Mouss'Protect" der Firma Purodor vertrieben (EG-Sicherheitsdatenblatt "Marly Skin Hautschutzschaum", überarbeitet: 06.07.2010 und Technisches Merkblatt "Mouss'Protect"). Zur Herstellung dieser beiden Schäume sind Treibmittel, insbesondere Alkane wie Propan und Butan, notwendig. Zudem enthalten diese Hautschutzschäume Siliconverbindungen als Schutzfilmbildner. Nachteilig bei diesen Schäumen ist der Einsatz des Treibmittels, zum einen aus Gründen der Ressourcenschonung und des Umweltschutzes, zum anderen, da die Treibmittel in einem Druckbehälter aufbewahrt werden müssen, was ein gegenüber drucklosen Behältern erhöhtes sicherheitstechnisches Risiko darstellt. Zudem ist der Einsatz von Siliconverbindungen häufig unerwünscht.

Der Hautschutzschaum "C60" der Orochemie benötigt keine Treibmittel zur Herstellung, allerdings enthält er ebenfalls eine Siliconverbindung (Produktinformation "C60 Hautschutzschaum", Stand August 2015).

Siliconverbindungen sind häufig - insbesondere in der Automobilindustrie und bei den entsprechenden Zulieferindustrien - unerwünscht, da sie bei Materialkontakt zu Benetzungsstörungen führen und so Lackier- und Beschichtungsvorgänge beeinträchtigen können.

Es besteht daher Bedarf an weiteren Präparaten zum Hautschutz, die die vorstehend beschriebenen Nachteile des Standes der Technik nicht oder in vermindertem Maße aufweisen.

Gelöst wurde diese Aufgabe durch eine wässrige Lösung (wL) enthaltend die Komponenten (A) bis (E), mit den Mengenangaben entsprechend Anspruch 1:
(A) Wasser,
(B) mindestens ein Tensid, das ausgewählt ist aus der Gruppe bestehend aus amphoteren Tensiden, anionischen Tensiden und nichtionischen Tensiden,
(C) mindestens ein Fluorpolymer,
(D) mindestens einen ersten pH-Regulator, der basisch ist, und
(E) mindestens einen zweiten pH-Regulator, der sauer ist.

Es wurde überraschend gefunden, dass die erfindungsgemäße wässrige Lösung (wL) sehr leicht herstellbar ist und gute Hautschutzeigenschaften besitzt, insbesondere ein gutes Einziehverhalten und eine gute Hautverträglichkeit, insbesondere eine gute Akzeptanz.

Die Hautschutzeigenschaften wurden mithilfe des Bovine Udder Skin Modells (BUS-Modell, Pittermann W., Jackwerth B. and Schmitt M. (1997): The isolated perfused bovine udder skin model. A new in-vitro model for the assessment of skin penetration and irritation. In-vitro Toxicology 10, pp 17-21) anhand von Modellschadstoffen (wasserlöslich und nicht wasserlöslich) ermittelt.

Insbesondere bei Anwendung der wässrigen Lösung als wässriger Schaum ist eine gute Verteilung und eine exakte Darreichungsform möglich, woraus eine besonders genaue Dosierung resultiert. Es werden nur geringe Fingerabdrücke erzeugt, sodass die wässrige Lösung, insbesondere der wässrige Schaum, abdruckarm oder sogar weitgehend abdruckfrei ist.

Es wurde außerdem überraschend gefunden, dass mit der wässrigen Lösung (wL) ein wässriger Schaum (wS) hergestellt werden kann, wobei druckbehälterfreie Systeme eingesetzt werden können und kein Treibgas (Treibmittel) oder Druckluft eingesetzt werden muss zur Herstellung des wässrigen Schaums (wS). Dies ist insbesondere sicherheitstechnisch vorteilhaft und besonders umweltschonend.

Bei Einsatz der wässrigen Lösung (wL) in einem wässrigen Schaum (wS) ist zudem die Überdosierungsgefahr minimiert. Dadurch kann die Akzeptanz beim Anwender erhöht werden, wodurch wiederum die flächendeckende Anwendung insbesondere im Bereich des betrieblichen Hautschutzes verbessert werden kann. Durch die leicht zu erreichende gleichmäßige Verteilung des Produktes können alle Hautstellen mit dem Hautschutzpräparat benetzt und so geschützt werden.

Vorteilhaft ist zudem, dass die wässrige Lösung (wL) einen kaum spürbaren Film auf der Haut hinterlässt.

Nachfolgend wird die vorliegende Erfindung näher erläutert.

Erfindungsgemäß enthält die wässrige Lösung (wL) als Komponente (A) Wasser, als Komponente (B) mindestens ein Tensid, das ausgewählt ist aus der Gruppe bestehend aus amphoteren Tensiden, anionischen Tensiden und nichtionischen Tensiden, als Komponente (C) mindestens ein Fluorpolymer, als Komponente (D) mindestens einen ersten pH-Regulator, der basisch ist, und als Komponente (E) mindestens einen zweiten pH-Regulator, der sauer ist.

Im Rahmen der vorliegenden Erfindung werden die Begriffe "Komponente (A)" und "Wasser" synonym gebraucht und besitzen daher die gleiche Bedeutung. Entsprechendes gilt für die Begriffe "Komponente (B)" und "mindestens ein Tensid", "Komponente (C)" und "mindestens ein Fluorpolymer", "Komponente (D)" und "mindestens ein erster pH-Regulator" sowie "Komponente (E)" und "mindestens ein zweiter pH-Regulator". Diese Begriffe werden ebenfalls jeweils synonym gebraucht und besitzen daher die gleiche Bedeutung.

"Mindestens ein Tensid" bedeutet im Rahmen der vorliegenden Erfindung sowohl genau ein Tensid als auch eine Mischung aus zwei oder mehreren Tensiden. Bevorzugt ist genau ein Tensid oder eine Mischung aus genau zwei Tensiden.

"Mindestens ein Fluorpolymer" bedeutet im Rahmen der vorliegenden Erfindung sowohl genau ein Fluorpolymer als auch eine Mischung aus zwei oder mehreren Fluorpolymeren.

"Mindestens ein erster pH-Regulator" bedeutet im Rahmen der vorliegenden Erfindung sowohl genau ein erster pH-Regulator als auch eine Mischung aus zwei oder mehreren ersten pH-Regulatoren.

"Mindestens ein zweiter pH-Regulator" bedeutet im Rahmen der vorliegenden Erfindung sowohl genau ein zweiter pH-Regulator als auch eine Mischung aus zwei oder mehreren zweiten pH-Regulatoren.

Die beanspruchte wässrige Lösung (wL) enthält Inhaltsstoffe im Bereich von 70 bis 98 Gew.-% der Komponente (A), im Bereich von 0,1 bis 10 Gew.-% der Komponente (B), im Bereich von 0,1 bis 10 Gew.-% der Komponente (C), im Bereich von 0,01 bis 5 Gew.-% der Komponente (D) und im Bereich von 0,01 bis 5 Gew.-% der Komponente (E), jeweils bezogen auf die Summe der Gewichtsprozente der Komponenten (A) bis (E), bevorzugt bezogen auf das Gesamtgewicht der wässrigen Lösung (wL).

Besonders bevorzugt enthält die wässrige Lösung (wL) im Bereich von 76 bis 95 Gew.-% der Komponente (A), im Bereich von 0,5 bis 8 Gew.-% der Komponente (B), im Bereich von 0,5 bis 8 Gew.-% der Komponente (C), im Bereich von 0,5 bis 8 Gew.-% der Komponente (C), im Bereich von 0,03 bis 4 Gew.-% der Komponente (D) und im Bereich von 0,03 bis 4 Gew.-% der Komponente (E), jeweils bezogen auf die Summe der Gewichtsprozente der Komponenten (A) bis (E), bevorzugt bezogen auf das Gesamtgewicht der wässrigen Lösung (wL).

Insbesondere bevorzugt enthält die wässrige Lösung (wL) im Bereich von 84 bis 92 Gew.-% der Komponente (A), im Bereich von 1 bis 6 Gew.-% der Komponente (B), im Bereich von 1 bis 6 Gew.-% der Komponente (C), im Bereich von 0,05 bis 2 Gew.-% der Komponente (D) und im Bereich von 0,05 bis 2 Gew.-% der Komponente (E), jeweils bezogen auf die Summe der Gewichtsprozente der Komponenten (A) bis (E), bevorzugt bezogen auf das Gesamtgewicht der wässrigen Lösung (wL).

Es ist darüber hinaus bevorzugt, dass das Gewichtsverhältnis der Komponente (A) zur Komponente (B) in der wässrigen Lösung (wL) im Bereich von 10 : 1 bis 200 : 1 liegt, bevorzugt im Bereich von 50 : 1 bis 100 : 1, insbesondere bevorzugt im Bereich von 60 : 1 bis 80 : 1.

Erfindungsgemäß ist die Komponente (A) der erfindungsgemäßen wässrigen Lösung (wL) Wasser. Als Wasser kann jedes dem Fachmann bekannte Wasser eingesetzt werden, das für die Herstellung kosmetischer Mittel geeignet ist. Vorzugsweise ist das Wasser ausgewählt aus der Gruppe bestehend aus Trinkwasser, enthärtetem Trinkwasser, destilliertem Wasser, deionisiertem Wasser und Mischungen daraus. Insbesondere bevorzugt wird destilliertes und/oder deionisiertes Wasser eingesetzt.

Bevorzugt enthält die erfindungsgemäße wässrige Lösung (wL) zusätzlich zumindest eine der Komponenten
(F) mindestens einen wasserlöslichen Pflegestoff und/oder
(G) mindestens einen Duftstoff und/oder
(H) mindestens einen Konservierungsstoff und/oder
(I) mindestens einen zusätzlichen Schutzfilmbildner.

Im Rahmen der vorliegenden Erfindung werden die Begriffe "Komponente (F)" und "mindestens ein wasserlöslicher Pflegestoff" synonym gebraucht und besitzen daher die gleiche Bedeutung. Entsprechendes gilt für die Begriffe "Komponente (G)" und "mindestens ein Duftstoff", "Komponente (H)" und "mindestens einen Konservierungsstoff" sowie "Komponente (I)" und "mindestens ein zusätzlicher Schutzfilmbildner". Sie werden im Rahmen der vorliegenden Erfindung ebenfalls jeweils synonym gebraucht und besitzen daher die gleiche Bedeutung.

"Mindestens ein wasserlöslicher Pflegestoff" bedeutet im Rahmen der vorliegenden Erfindung sowohl genau ein wasserlöslicher Pflegestoff als auch eine Mischung aus zwei oder mehreren wasserlöslichen Pflegestoffen.

"Mindestens ein Duftstoff" bedeutet im Rahmen der vorliegenden Erfindung sowohl genau ein Duftstoff als auch eine Mischung aus zwei oder mehreren Duftstoffen.

"Mindestens ein Konservierungsstoff" bedeutet im Rahmen der vorliegenden Erfindung sowohl genau ein Konservierungsstoff als auch eine Mischung aus zwei oder mehreren Konservierungsstoffen.

"Mindestens ein zusätzlicher Schutzfilmbildner" bedeutet im Rahmen der vorliegenden Erfindung sowohl genau ein zusätzlicher Schutzfilmbildner als auch eine Mischung aus zwei oder mehreren zusätzlichen Schutzfilmbildnern.

Die Komponenten (F) bis (I) werden ebenso wie die vorstehend genannten Komponenten (B) bis (E) weiter unten näher beschrieben.

Enthält die wässrige Lösung (wL) zusätzlich die Komponente (F), so enthält die wässrige Lösung (wL) bevorzugt im Bereich von 0,1 bis 10 Gew.-%, besonders bevorzugt im Bereich von 0,5 bis 8 Gew.-%, insbesondere bevorzugt im Bereich von 1 bis 5 Gew.-%, der Komponente (F), jeweils bezogen auf die Summe der Gew.-% der Komponenten (A) bis (I), bevorzugt bezogen auf das Gesamtgewicht der wässrigen Lösung (wL).

Enthält die wässrige Lösung (wL) zusätzlich die Komponente (G), so enthält die wässrige Lösung (wL) bevorzugt im Bereich von 0,02 bis 2 Gew.-%, besonders bevorzugt im Bereich von 0,05 bis 1 Gew.-%, insbesondere bevorzugt im Bereich von 0,1 bis 1 Gew.-%, der Komponente (G), jeweils bezogen auf die Summe der Gew.-% der Komponenten (A) bis (I), bevorzugt bezogen auf das Gesamtgewicht der wässrigen Lösung (wL).

Enthält die wässrige Lösung (wL) zusätzlich die Komponente (H), so enthält die wässrige Lösung (wL) bevorzugt im Bereich von 0,001 bis 2 Gew.-%, besonders bevorzugt im Bereich von 0,01 bis 1,5 Gew.-%, insbesondere bevorzugt im Bereich von 0,1 bis 1 Gew.-%, der Komponente (H), jeweils bezogen auf die Summe der Gew.-% der Komponenten (A) bis (I), bevorzugt bezogen auf das Gesamtgewicht der wässrigen Lösung (wL).

Enthält die wässrige Lösung (wL) zusätzlich die Komponente (I), so enthält die wässrige Lösung (wL) bevorzugt im Bereich von 0,1 bis 5 Gew.-%, besonders bevorzugt im Bereich von 0,5 bis 4 Gew.-%, insbesondere bevorzugt im Bereich von 1 bis 3 Gew.-%, der Komponente (I), jeweils bezogen auf die Summe der Gew.-% der Komponenten (A) bis (I), bevorzugt bezogen auf das Gesamtgewicht der wässrigen Lösung (wL).

Enthält die erfindungsgemäße wässrige Lösung (wL) zusätzlich die Komponenten (F), (G), (H) und (I), so ist es bevorzugt, dass die wässrige Lösung zusätzlich im Bereich von 0,1 bis 10 Gew.-% der Komponente (F), 0,02 bis 2 Gew.-% der Komponente (G), 0,001 bis 2 Gew.-% der Komponente (H) und 0,1 bis 5 Gew.-% der Komponente (I) enthält, jeweils bezogen auf die Summe der in der wässrigen Lösung (wL) enthaltenen Komponenten (A) bis (I), bevorzugt bezogen auf das Gesamtgewicht der wässrigen Lösung (wL).

Bevorzugt enthält die wässrige Lösung (wL) zusätzlich im Bereich von 0,5 bis 8 Gew.-% der Komponente (F), im Bereich von 0,05 bis 1 Gew.-% der Komponente (G), im Bereich von 0,01 bis 1,5 Gew.-% der Komponente (H) und im Bereich von 0,5 bis 4 Gew.-% der Komponente (I), jeweils bezogen auf die Summe der Gewichtsprozente der in der wässrigen Lösung (wL) enthaltenen Komponenten (A) bis (I), bevorzugt bezogen auf das Gesamtgewicht der wässrigen Lösung (wL).

Besonders bevorzugt enthält die wässrige Lösung (wL) zusätzlich im Bereich von 1 bis 5 Gew.-% der Komponente (F), im Bereich von 0,1 bis 1 Gew.-% der Komponente (G), im Bereich von 0,1 bis 1 Gew.-% der Komponente (H) und im Bereich von 1 bis 3 Gew.-% der Komponente (I), jeweils bezogen auf die Summe der Gewichtsprozente der in der wässrigen Lösung (wL) enthaltenen Komponenten (A) bis (I), bevorzugt bezogen auf das Gesamtgewicht der wässrigen Lösung (wL).
Üblicherweise addieren sich die Gewichtsprozente der in der wässrigen Lösung (wL) enthaltenen Komponenten (A) bis (E) sowie der gegebenenfalls zusätzlich in der wässrigen Lösung (wL) enthaltenen Komponenten (F) bis (I) zu 100 %.
Es versteht sich von selbst, dass, wenn die wässrige Lösung (wL) zusätzlich zumindest eine der Komponenten (F) bis (I) enthält, sich die Gewichtsprozente der Komponenten (A) bis (E), insbesondere der Komponente (A), entsprechend reduzieren können. Beispielsweise liegt die Untergrenze der Gewichtsprozente der Komponente (A) dann bei 40 Gew.-%, bevorzugt bei 50 Gew.-%, besonders bevorzugt bei 60 Gew.-%, jeweils bezogen auf die Summe der Gewichtsprozente der in der wässrigen Lösung (wL) enthaltenen Komponenten (A) bis (I), bevorzugt bezogen auf das Gesamtgewicht der wässrigen Lösung (wL).

Die erfindungsgemäße wässrige Lösung (wL) enthält als Komponente (A) Wasser. Die Komponente (A) dient daher als Lösungsmittel in der wässrigen Lösung (wL). Die weiteren in der wässrigen Lösung (wL) enthaltenen Komponenten (B) bis (E) sowie die gegebenenfalls zusätzlich enthaltenen Komponenten (F) bis (I) sind in dem Lösungsmittel Wasser, also in der Komponente (A), gelöst. Die Komponenten (B) bis (I) bilden daher mit dem Wasser zusammen eine homogene Phase.

Die erfindungsgemäße wässrige Lösung (wL) ist also keine Dispersion, insbesondere keine Emulsion und keine Suspension.

Unter einer Dispersion wird im Rahmen der vorliegenden Erfindung ein heterogenes Gemisch aus zumindest zwei verschiedenen Komponenten verstanden. Die Komponenten sind nicht mischbar.

Unter einer Suspension wird im Rahmen der vorliegenden Erfindung ein heterogenes Gemisch aus zumindest zwei verschiedenen Komponenten verstanden, wobei eine Komponente flüssig und die andere Komponente fest vorliegt. Es versteht sich von selbst, dass die flüssige Komponente auch ein homogenes Gemisch aus zwei oder mehreren Komponenten sein kann. Entsprechend kann die feste Komponente auch ein homogenes oder heterogenes Gemisch aus zwei oder mehreren Komponenten sein.

Unter einer Emulsion wird im Rahmen der vorliegenden Erfindung ein heterogenes Gemisch aus zumindest zwei verschiedenen Komponenten verstanden, wobei jede der zumindest zwei Komponenten flüssig vorliegt. Es versteht sich von selbst, dass die eine flüssige Komponente auch ein homogenes Gemisch aus zwei oder mehreren Komponenten sein kann. Entsprechend kann die andere flüssige Komponente ebenfalls auch ein homogenes Gemisch aus zwei oder mehreren Komponenten sein.

Nachfolgend werden die in der wässrigen Lösung (wL) enthaltenen Komponenten (B) bis (E) sowie die gegebenenfalls zusätzlich enthaltenen Komponenten (F) bis (I) näher erläutert.
Erfindungsgemäß ist die Komponente (B) mindestens ein Tensid.

Tenside sind dem Fachmann als solche bekannt. Sie weisen im Molekül üblicherweise einen unpolaren und einen polaren Teil auf. Sie umfassen daher im Allgemeinen einen hydrophoben, also wasserabweisenden, Kohlenwasserstoffrest und einen hydrophilen Molekülteil.

Die Komponente (B) ist wasserlöslich.

Erfindungsgemäß ist das mindestens eine Tensid ausgewählt aus der Gruppe bestehend aus amphoteren Tensiden, anionischen Tensiden und nichtionischen Tensiden. Bevorzugt ist das mindestens eine Tensid ausgewählt aus der Gruppe bestehend aus amphoteren Tensiden und anionischen Tensiden, besonders bevorzugt aus der Gruppe bestehend aus amphoteren Tensiden.

In einer Ausführungsform der vorliegenden Erfindung ist es bevorzugt, dass die wässrige Lösung (wL) kein nichtionisches Tensid enthält.

"Kein nichtionisches Tensid" bedeutet im Rahmen der vorliegenden Erfindung weniger als 0,5 Gew.-%, bevorzugt weniger als 0,1 Gew.-%, insbesondere bevorzugt weniger als 0,01 Gew.-% nichtionisches Tensid in der wässrigen Lösung (wL), bezogen auf das Gesamtgewicht der wässrigen Lösung (wL).

Amphotere Tenside, auch ampholytische Tenside genannt, sind Tenside, die sowohl eine negativ geladene funktionelle Gruppe als auch eine positiv geladene funktionelle Gruppe aufweisen. Es versteht sich von selbst, dass sich die negativ geladene funktionelle Gruppe und die positiv geladene funktionelle Gruppe auch erst in Lösung, insbesondere in Lösung in der Komponente (A), bilden können.

Geeignete amphotere Tenside sind beispielsweise ausgewählt aus der Gruppe bestehend aus Cocamidopropylbetain, Kokosbetain, Natriumkokosfettsäureamphoacetat.

Anionische Tenside sind Tenside, die eine oder mehrere negativ geladene funktionelle Gruppen aufweisen. Derartige negativ geladene funktionelle Gruppen sind dem Fachmann bekannt und beispielsweise Carboxylatgruppen (-COO⁻), Sulfonatgruppen (-SO₃⁻), Sulfatgruppen (-SO₄²⁻) oder Phosphatgruppen (-PO₄³⁻).

Dem Fachmann ist klar, dass anionische Tenside üblicherweise erst in Lösung, insbesondere in dem als Komponente (A) eingesetzten Wasser, dissoziieren unter Erhalt der negativ geladenen funktionellen Gruppe.

Als anionische Tenside eignen sich alle dem Fachmann bekannten anionischen Tenside. Bevorzugt sind anionische Tenside, ausgewählt aus der Gruppe bestehend aus Dinatriumlaurylethersulfosuccinat, Dinatriumlaurylsulfosuccinat, Natriumlaurylsulfat, Natriumlaurylethersulfat, Natriumkokosfettsäuresulfat, MIPA-Laurylethersulfat, MEA-Laurylsulfat, Natriumlauroylglutamat, Dinatriumcocoylglutamat und Natriumlaurylsulfoacetat.

Nichtionische Tenside weise keine negativ oder positiv geladenen funktionellen Gruppen auf und dissoziieren auch in Wasser nicht. Als nichtionische Tenside eigenen sich alle dem Fachmann bekannten nichtionischen Tenside, die wasserlöslich sind.

Bevorzugt sind nichtionische Tenside, ausgewählt aus der Gruppe bestehend aus Kokosglucosid, Laurylglucosid, Decylglucosid, Caprylylglukosid, Caprylyl / Capryl Glucosid, Kokosfettsäurediethanolamid und Sojafettsäurediethanolamid.

Es ist daher erfindungsgemäß bevorzugt, dass das mindestens eine Tensid (B) ausgewählt ist aus der Gruppe bestehend aus Cocamidopropylbetain, Dinatriumlaurylethersulfosuccinat, Kokosbetain, Natriumkokosfettsäureamphoacetat, Dinatriumlaurylsulfosuccinat, Natriumlaurylsulfat, Natriumlaurylethersulfat, Natriumkokosfettsäuresulfat, MIPA-Laurylethersulfat, MEA-Laurylsulfat, Natriumlauroylglutamat, Dinatriumcocoylglutamat, Natriumlaurylsulfoacetat, Kokosglucosid, Laurylglucosid, Decylglucosid, Caprylylglukosid, Caprylyl/Capryl-Glucosid, Kokosfettsäurediethanolamid und Sojafettsäurediethanolamid.

Cocamidopropylbetain ist auch bekannt unter dem INCI-Namen COCAMIDOPROPYL BETAINE und unter dem IUPAC-Namen {[3-(Dodecanoylamino)propyl](dimethyl)ammonium}acetat. Es trägt die CAS-Nummer 61789-40-0.

Dinatriumlaurylethersulfosuccinat ist auch bekannt unter dem INCI-Namen DISODIUM LAURETH SULFOSUCCINATE und unter dem IUPAC-Namen Dinatrium-4-[2-[2-(2-dodecyloxy)ethoxy]ethoxy]ethoxy]-4-oxo-2-sulfonatobutanoat.

Kokosbetain ist auch bekannt unter dem INCI-Namen COCO-BETAINE. Es trägt die CAS-Nummer 68424-94-2.

Natriumkokosfettsäureamphoacetat ist auch bekannt unter dem INCI-Namen SODIUM COCOAMPHOACETATE. Es trägt die CAS-Nummer 90387-76-1.

Dinatriumlaurylsulfosuccinat ist auch bekannt unter dem INCI-Namen DISODIUM LAURYL SULFOSUCCINATE. Es trägt die CAS-Nummern 13192-12-6 / 19040-44-9 / 26838-05-1.

Natriumlaurylsulfat ist auch bekannt unter dem INCI-Namen SODIUM LAURYL SULFATE und unter dem IUPAC-Namen Natriumdodecylsulfat. Es trägt die CAS-Nummer 151-21-3.

Natriumlaurylethersulfat ist auch bekannt unter dem INCI-Namen SODIUM LAURETH SULFATE. Es trägt die CAS-Nummern 3088-31-1 / 9004-82-4 / 68891-38-3 / 1335-72-4 / 68585-34-2 / 91648-56-5.

Natriumkokosfettsäuresulfat ist auch bekannt unter dem INCI-Namen SODIUM COCO-SULFATE. Es trägt die CAS-Nummer 97375-27-4.

MIPA-Laurylethersulfat ist auch bekannt unter dem INCI-Namen MIPA-LAURETH SULFATE. Es trägt die CAS-Nummern 83016-76-6 / 9062-04-8.

MEA-Laurylsulfat ist auch bekannt unter dem INCI-Namen MEA-LAURYL SULFATE und unter dem IUPAC-Namen (2-Hydroxyethyl)ammoniumdodecylsulfat. Es trägt die CAS-Nummer 4722-98-9.

Natriumlauroylglutamat ist auch bekannt unter dem INCI-Namen SODIUM LAUROYL GLU-TAMAT. Es trägt die CAS-Nummern 29923-31-7 / 29923-34-0 / 42926-22-7 / 98984-78-2.

Dinatriumcocoylglutamat ist auch bekannt unter dem INCI-Namen DISODIUM COCOYL GLUTAMATE. Es trägt die CAS-Nummer 68187-30-4.

Natriumlaurylsulfoacetat ist auch bekannt unter dem INCI-Namen SODIUM LAURYL SULFOACETATE und unter dem IUPAC-Namen Natrium 2-(dodecyloxy)-2-oxoethan-1-sulfonat. Es trägt die CAS-Nummer 1847-58-1.

Kokosglucosid ist auch bekannt unter dem INCI-Namen COCO GLUCOSIDE. Es trägt die CAS-Nummern 110615-47-9 und 68515-73-1.

Laurylglucosid ist auch bekannt unter dem INCI-Namen LAURYL GLUCOSIDE. Es trägt die CAS-Nummer 110615-47-9.

Decylglucosid ist auch bekannt unter dem INCI-Namen DECYL GLUCOSIDE. Es trägt die CAS-Nummern 54549-25-6, 58846-77-8, 141464-42-8, 68515-73-1.

Caprylylglukosid ist auch bekannt unter dem INCI-Namen CAPRYLYL GLUCOSIDE. Es trägt die CAS-Nummer 29836-26-8.

Caprylyl/Capryl-Glucosid ist auch bekannt unter dem INCI-Namen CAPRYLYL/CAPRYL GLUCOSIDE. Es trägt die CAS-Nummer 68515-73-1.

Kokosfettsäurediethanolamid ist auch bekannt unter dem INCI-Namen COCAMIDE DEA. Es trägt die CAS-Nummer 68603-42-9.

Sojafettsäurediethanolamid ist auch bekannt unter dem INCI-Namen SOYAMIDE DEA. Es trägt die CAS-Nummer 68425-47-8

Unter dem INCI-Namen wird der "International Nomenclature of Cosmetic Ingredients"-Name verstanden. Er wird international zur Deklaration kosmetischer Inhaltsstoffe verwendet. Namen und zugehörige Daten sind in der Datenbank Coslng der EU (http://ec.europa.eu/growth/tools-databases/cosing/, abgerufen am 17. März 2016) hinterlegt. Der INCI-Name ist dem Fachmann als solcher geläufig. Entsprechendes gilt für den IUPAC-Namen sowie die CAS-Nummer.

Es versteht sich von selbst, dass im Rahmen der vorliegenden Erfindung sämtliche Bezeichnungen der beschriebenen Komponenten, also der INCI-Name, der IUPAC-Name sowie der Trivialname synonym gebraucht werden und daher die gleiche Bedeutung besitzen.

Die Komponente (C) ist mindestens ein Fluorpolymer.

Unter Fluorpolymeren werden im Rahmen der vorliegenden Erfindung von organischen Monomeren abgeleitete Polymere verstanden, bei denen zumindest ein Wasserstoffatom durch Fluor ersetzt worden ist. Erfindungsgemäß bevorzugt sind perfluorierte Polymere. Unter perfluorierten Polymeren werden Polymere verstanden, bei denen alle Wasserstoffatome durch Fluoratome ersetzt worden sind sowie die daraus abgeleiteten Derivate und Copolymere. Insbesondere bevorzugt ist das mindestens eine Fluorpolymer (C), ausgewählt aus Perfluorpolyethern, Polyurethan-26 und Polyurethan-27, besonders bevorzugt aus Perfluorpolyetherphosphaten und insbesondere bevorzugt aus Perfluorethoxymethoxy-difluorethyl-polyethylenglycolphosphat.

Perfluorpolyether sind Polyether, bei denen die Wasserstoffatome durch Fluoratome ersetzt worden sind sowie die daraus abgeleiteten Derivate und Copolymere. Perfluorpolyetherphosphate sind Derivate der Perfluorpolyether, bei denen die endständigen OH-Gruppen ganz oder teilweise mit Phosphorsäure verestert worden sind.

Perfluorethoxymethoxy-difluorethyl-polyethylenglycolphosphat wird hergestellt durch oxidative Photopolymeristation von Tetrafluorethylen und anschließende Veresterung, Reduktion, Ethoxylierung und Phosphatierung. Das mittlere Molekulargewicht beträgt etwa 2500. Es ist unter dem INCI-Namen POLYPERFLUOROETHOXYMETHOXY DIFLUOROETHYL PEG PHOSPHATE bekannt und trägt die CAS-Nummer 200013-65-6.

Polyurethan-26 ist auch bekannt unter dem INCI-Namen POLYURETHANE-26. Es trägt die CAS-Nummer 328389-90-8.

Polyurethan-27 ist auch bekannt unter dem INCI-Namen POLYURETHANE-27. Es trägt die CAS-Nummer 328389-91-9.

Die Komponente (C) dient als Schutzfilmbildner. Unter Schutzfilmbildnern werden im Rahmen der vorliegenden Erfindung Komponenten, insbesondere Polymere, verstanden, die auf der Anwendungsfläche, also beispielsweise auf der Haut oder auf den Haaren, insbesondere auf der Haut, einen Film ausbilden, der den direkten, unerwünschten Kontakt von Stoffen mit der Anwendungsfläche unterbindet oder vermindert und/oder der das unerwünschte Eindringen von Stoffen in die Anwendungsfläche unterbindet oder vermindert.

Die Komponente (D) ist erfindungsgemäß mindestens ein erster pH-Regulator, der basisch ist.

Unter "basisch" wird im Rahmen der vorliegenden Erfindung verstanden, dass der erste pH-Regulator, wenn er in einer Konzentration von 0,1 mol/l in Wasser gelöst wird, einen pH-Wert von > 7, bevorzugt von > 9 und insbesondere bevorzugt von > 11, aufweist.

Geeignete erste pH-Regulatoren sind dem Fachmann als solche bekannt. Vorzugsweise ist der erste pH-Regulator (D) ausgewählt aus der Gruppe bestehend aus Natriumhydroxid, Kaliumhydroxid, Natriumcarbonat, Kaliumcarbonat, Trinatriumorthophosphat, Natriumtriphosphat und Kaliumtriphosphat.

Natriumhydroxid ist auch unter dem INCI-Namen SODIUM HYDROXIDE bekannt und trägt die CAS-Nummer 1310-73-2.

Kaliumhydroxid ist auch unter dem INCI-Namen POTASSIUM HYDROXIDE bekannt und trägt die CAS-Nummer 1310-58-3.

Natriumcarbonat ist auch unter dem INCI-Namen SODIUM CARBONATE bekannt und trägt die CAS-Nummer 497-19-8.

Kaliumcarbonat ist auch unter dem INCI-Namen POTASSIUM CARBONATE bekannt und trägt die CAS-Nummer 584-08-7.

Trinatriumorthophosphat ist auch unter dem INCI-Namen TRISODIUM PHOSPHATE bekannt und trägt die CAS-Nummer 7601-54-9.

Natriumtriphosphat ist auch unter dem INCI-Namen PENTASODIUM TRIPHOSPHATE bekannt und trägt die CAS-Nummer 7758-29-4.

Kaliumtriphosphat ist auch unter dem INCI-Namen PENTAPOTASSIUM TRIPHOSPHATE bekannt und trägt die CAS-Nummer 13845-36-8.

Die Komponente (E) ist mindestens ein zweiter pH-Regulator, der sauer ist.

Unter "sauer" wird im Rahmen der vorliegenden Erfindung verstanden, dass der zweite pH-Regulator gelöst in einer Konzentration von 0,1 mol/l in Wasser einen pH-Wert von < 7, bevorzugt von < 5 , insbesondere bevorzugt von < 3, aufweist.

Derartige zweite pH-Regulatoren sind dem Fachmann als solche bekannt. Bevorzugt ist der zweite pH-Regulator (E) ausgewählt aus der Gruppe bestehend aus Citronensäure, Milchsäure, Essigsäure, Ascorbinsäure, Phosphorsäure, Adipinsäure, Bernsteinsäure und Salzsäure.

Citronensäure ist als wasserfreies Produkt und als Monohydrat gebräuchlich. Sie ist auch bekannt unter dem INCI-Namen CITRIC ACID und unter den IUPAC-Namen 2-Hydroxy-1,2,3-propantricarbonsäure bzw. 2-Hydroxy-1,2,3-propan-tricarbonsäure Monohydrat Sie trägt die CAS-Nummern 77-92-9 / 5949-29-1.

Milchsäure ist auch bekannt unter dem INCI-Namen LACTIC ACID. Ihr IUPAC-Name lautet 2-Hydroxypropansäure und sie trägt die CAS-Nummer 50-21-5.

Essigsäure ist unter dem INCI-Namen ACETIC ACID bekannt und trägt den IUPAC-Namen Ethansäure. Ihre CAS-Nummer lautet 64-19-7.

Phosphorsäure ist auch bekannt unter dem INCI-Namen PHOSPHORIC ACID und trägt den IUPAC-Namen Phosphorsäure. Sie trägt die CAS-Nummer 7664-38-2.

Adipinsäure trägt den INCI-Namen ADIPIC ACID und den IUPAC-Namen 1,4-Butandicarbonsäure. Ihre CAS-Nummer lautet 124-04-9.

Bernsteinsäure ist auch bekannt unter dem INCI-Namen SUCCINIC ACID und dem IUPAC-Namen 1,2-Ethandicarbonsäure. Sie trägt die CAS-Nummer 110-15-6.

Salzsäure ist auch bekannt unter dem INCI-Namen HYDROCHLORIC ACID und dem IUPAC-Namen Chlorwasserstoff. Sie trägt die CAS-Nummer 7647-010.

Es ist erfindungsgemäß bevorzugt, dass die wässrige Lösung (wL) zusätzlich die Komponente (F), mindestens einen wasserlöslichen Pflegestoff, enthält. Derartige wasserlösliche Pflegestoffe sind dem Fachmann als solche bekannt. Unter Pflegestoffen werden Stoffe verstanden, die der Aufrechterhaltung oder Wiederherstellung der normalen Funktion der Haut, insbesondere der Epidermis und der Hornschichtbarriere dienen. Die Pflegestoffe können beispielsweise aus wasserbindenden Substanzen oder aus biochemisch aktiven Substanzen bestehen.

Bevorzugt ist der mindestens eine wasserlösliche Pflegestoff (F) ausgewählt aus der Gruppe bestehend aus Glycerin, Betain, Caprylylglykol, Dexpanthenol, Propylenglykol, Propandiol, Butylglykol, Hyaluronsäure, Natriumhyaluronat, Allantoin, Decylenglycol, Harnstoff, L-Arginin, Honig, Ascorbinsäure, Nikotinsäure, Nikotinsäureamid, Milchprotein, Collagen und wasserlöslichen, pflanzlichen Extrakten.

Unter wasserlöslichen, pflanzlichen Extrakten werden Stoffgemische verstanden, die durch Extraktion von Pflanzen, Pflanzenteilen oder Pflanzenerzeugnissen durch Extraktion mit polaren Lösungsmitteln hergestellt werden und die wasserlöslich sind. Diese sind dem Fachmann bekannt.

Glycerin ist auch bekannt unter dem INCI-Namen GLYCERIN und dem IUPAC-Namen Propan-1,2,3-triol. Es trägt die CAS-Nummer 56-81-5.

Betain ist auch bekannt unter dem INCI-Namen BETAINE und dem IUPAC-Namen N,N,N-Trimethylammonioacetat. Es trägt die CAS-Nummer 107-43-7.

Caprylylglykol ist auch bekannt unter dem unter dem INCI-Namen CAPRYLYL GLYCOL und dem IUPAC-Namen 1,2-Octandiol. Es trägt die CAS-Nummer 117-86-8.

Dexpanthenol ist auch bekannt unter dem INCI-Namen PANTHENOL und unter dem IUPC-Namen (2R)- 2,4-Dihydroxy-N-(3-hydroxypropyl)-3,3-dimethylbutyramid. Es trägt die CAS-Nummer 81-13-0.

Propylenglykol ist auch bekannt unter dem INCI-Namen PROPYLENE GLYCOL und unter dem IUPAC-Namen Propan- 1,2-diol. Es trägt die CAS-Nummer 57-55-6.

Propandiol ist auch bekannt unter dem INCI-Namen PROPANEDIOL und unter dem IUPAC-Namen Propan- 1,3-diol. Es trägt die CAS-Nummer 504-63-2.

Butylglykol ist auch bekannt unter dem INCI-Namen BUTYLENE GLYCOL und unter dem IUPAC-Namen Butan-1,3-diol. Es trägt die CAS-Nummer 107-88-0.

Hyaluronsäure ist auch bekannt unter dem INCI-Namen HYALURONIC ACID. Sie trägt die CAS-Nummer 9004-61-9.

Natriumhyaluronat ist auch bekannt unter dem INCI-Namen SODIUM HYALURONATE. Es trägt die CAS-Nummer 9067-32-7.

Allantoin ist auch bekannt unter dem INCI-Namen ALLANTOIN. Es trägt die CAS-Nummer 97-59-6.

Decylene Glycol ist auch bekannt unter dem INCI-Namen DECYLENE GLYCOLE und unter dem IUPAC-Namen 1,2-Dekandiol. Es trägt die CAS-Nummer 1119-86-4.

Harnstoff ist auch bekannt unter dem INCI-Namen UREA und unter dem IUPAC-Namen Diaminomethanal. Er trägt die CAS-Nummer 57-13-6.

L-Arginin ist auch bekannt unter dem INCI-Namen ARGININE. Es trägt die CAS-Nummer 74-79-3.

Honig ist auch bekannt unter dem INCI-Namen MEL.

Ascorbinsäure ist auch bekannt unter dem INCI-Namen ASCORBIC ACID und dem IUPAC-Namen (5R)-5-[(1S)-1,2-Dihydroxyethyl]-3,4-dihydroxy-5-hydrofuran-2-on und trägt die CAS-Nummer 50-81-7.

Nikotinsäure ist auch bekannt unter dem INCI-Namen NIACIN und unter dem IUPAC-Namen Pyridin- 3-carbonsäure. Sie trägt die CAS-Nummer 59-67-6.

Nikotinsäureamid ist auch bekannt unter dem INCI-Namen NIACINAMIDE und unter dem IUPAC-Namen Pyridin- 3-carboxamid. Es trägt die CAS-Nummer 98-92-0.

Milchprotein ist auch bekannt unter dem INCI-Namen MILK PROTEIN. Es trägt die CAS-Nummer 91053-68-8.

Collagen ist auch bekannt unter dem INCI-Namen COLLAGEN. Es trägt die CAS-Nummer 9007-34-8.

Darüber hinaus ist es bevorzugt, dass die wässrige Lösung (wL) zusätzlich mindestens einen Duftstoff (G) enthält.

Als mindestens ein Duftstoff (G) eignen sich alle dem Fachmann bekannten Duftstoffe, die zum Einsatz in wässrigen Lösungen und kosmetischen Mitteln geeignet sind. Als Duftstoffe sind sowohl geruchlich aktive Einzelstoffe als auch Gemische wie ätherische

Öle oder kosmetische Parfümöle geeignet. Kosmetische Parfümöle sind Zubereitungen aus geruchsaktiven Einzelstoffen und/oder Gemischen wie ätherischen Ölen sowie gegebenenfalls Lösungsmitteln und/oder weiteren Additiven. Sie werden unter der INCI-Bezeichnung PARFUM zusammengefasst und sind dem Fachmann bekannt.

Ebenso ist es bevorzugt, dass die wässrige Lösung (wL) zusätzlich mindestens einen Konservierungsstoff (H) enthält.

Unter Konservierungsstoffen werden im Rahmen der vorliegenden Erfindung Verbindungen verstanden, die in kosmetischen Mitteln ausschließlich oder überwiegend die Entwicklung von Mikroorganismen hemmen.

Als mindestens ein Konservierungsstoff eignen sich alle dem Fachmann bekannten Konservierungsstoffe, die für kosmetische Produkte zur Anwendung auf der Haut geeignet sind.

Bevorzugt ist der mindestens eine Konservierungsstoff (H) ausgewählt aus der Gruppe bestehend aus Phenoxyethanol, Benzylalkohol, Methylisothiazolinon, Chlormethylisothiazolinon, Dehydracetsäure, Natriumdehydracetat, Benzoesäure, Natriumbenzoat, Sorbinsäure, Kaliumsorbat, p-Hydroxybenzoesäuremethylester, p-Hydroxybenzoesäureethylester, p-Hydroxybenzoesäurepropylester, p-Hydroxybenzoesäurebutylester, Zinkpyrithion, Benzalkoniumchlorid, Bromnitropropandiol und Iodpropinylbutylcarbamat.

Phenoxyethanol ist auch bekannt unter dem INCI-Namen PHENOXYETHANOL und unter dem IUPAC-Namen 2-Phenoxyethanol. Es trägt die CAS-Nummer 122-99-6.

Benzylalkohol ist auch bekannt unter dem INCI-Namen BENZYL ALCOHOL und unter dem IUPAC-Namen Phenylmethanol. Es trägt die CAS-Nummer 100-51-6.

Methylisothiazolinon ist auch bekannt unter dem INCI-Namen METHYLISOTHIAZOLINONE und unter dem IUPAC-Namen 2-Methylisothiazol-3(2H)-on. Es trägt die CAS-Nummer 2682-20-4.

Chlormethylisothiazolinon ist auch bekannt unter dem INCI-Namen METHYLCHLOROISOTHIAZOLINONE und unter dem IUPAC-Namen 5-Chloro-2-methyl-4-isothiazolin-3-on. Es trägt die CAS-Nummer 26172-55-4.

Dehydracetsäure ist auch bekannt unter dem INCI-Namen DEHYDROACETIC ACID und unter dem IUPAC-Namen 3-Acetyl-2-hydroxy-6-methyl-4H-pyran-4-on. Es trägt die CAS-Nummer 520-45-6.

Natriumdehydracetat ist auch bekannt unter dem INCI-Namen SODIUM DEHYDROACETATE und unter dem IUPAC-Namen Natrium-1-(3,4-dihydro-6-methyl-2,4-dioxo-2H-pyran-3-yliden)-ethanolat. Es trägt die CAS-Nummer 4418-26-2.

Benzoesäure ist auch bekannt unter dem INCI-Namen BENZOIC ACID. Es trägt die CAS-Nummer 65-85-0.

Natriumbenzoat ist auch bekannt unter dem INCI-Namen SODIUM BENZOATE. Es trägt die CAS-Nummer 532-32-1.

Sorbinsäure ist auch bekannt unter dem INCI-Namen SORBIC ACID und unter dem IUPAC-Namen Hexa-2,4-diensäure. Es trägt die CAS-Nummer 110-44-1.

Kaliumsorbat ist auch bekannt unter dem INCI-Namen POTASSIUM SORBATE und unter dem IUPAC-Namen 2,4-Hexadiensäure, Kaliumsalz. Es trägt die CAS-Nummer 24634-61-5.

p-Hydroxybenzoesäuremethylester ist auch bekannt unter dem INCI-Namen METHYLPARABEN und unter dem IUPAC-Namen Methyl-4-hydroxybenzoat. Es trägt die CAS-Nummer 99-76-3.

p-Hydroxybenzoesäureethylester ist auch bekannt unter dem INCI-Namen ETHYLPARABEN und unter dem IUPAC-Namen Ethyl-4-hydroxybenzoat. Es trägt die CAS-Nummer 120-47-8.

p-Hydroxybenzoesäurepropylester ist auch bekannt unter dem INCI-Namen PROPYLPARABEN und unter dem IUPAC-Namen Propyl-4-hydroxybenzoat. Es trägt die CAS-Nummer 94-13-3.

p-Hydroxybenzoesäurebutylester ist auch bekannt unter dem INCI-Namen BUTYLPARABEN und unter dem IUPAC-Namen Butyl-4-hydroxybenzoat. Es trägt die CAS-Nummer 94-26-8.

Zinkpyrithion ist auch bekannt unter dem INCI-Namen ZINC PYRITHIONE und unter dem IUPAC-Namen Bis[1-hydroxy-2(1H)-pyridin-thionato] Zink. Es trägt die CAS-Nummer 13463-41-7.

Benzalkoniumchlorid ist auch bekannt unter dem INCI-Namen BENZALKONIUM CHLORIDE. Es trägt die CAS-Nummer 8001-54-5.

Bromnitropropandiol ist auch bekannt unter dem INCI-Namen 2-BROMO-2-NITROPROPANE-1,3-DIOL und unter dem IUPAC-Namen 2-Brom-2-nitropropan-1,3-diol. Es trägt die CAS-Nummer 52-51-7.

lodpropinylbutylcarbamat ist auch bekannt unter dem INCI-Namen IODOPROPYNYL BUTYLCARBAMATE und unter dem IUPAC-Namen 3-lod-2-propinylbutylcarbamat. Es trägt die CAS-Nummer 55406-53-6.

Es ist erfindungsgemäß weiterhin bevorzugt, dass die wässrige Lösung (wL) zusätzlich mindestens einen zusätzlichen Schutzfilmbildner (I) enthält.

Unter Schutzfilmbildnern werden im Rahmen der vorliegenden Erfindung Verbindungen, insbesondere Polymere, verstanden, die in der Lage sind, auf der Anwendungsfläche, beispielsweise auf der Haut oder den Haaren, insbesondere auf der Haut, einen Film auszubilden, der den direkten, unerwünschten Kontakt mit Stoffen mit der Anwendungsfläche unterbindet oder vermindert oder der das unerwünschte Eindringen von Stoffen in die Anwendungsfläche unterbindet oder vermindert.

Geeignete zusätzliche Schutzfilmbildner sind dem Fachmann als solche bekannt. Bevorzugt ist der mindestens eine zusätzliche Schutzfilmbildner (I) ausgewählt aus der Gruppe bestehend aus Polyvinylalkoholen, Cellulosederivaten, Polyacrylaten, Polyvinylpyrrolidonen, Chitosanderivaten, Polyestern, Polyamiden, Polyurethanen und Polystyrolderivaten .

Besonders bevorzugt ist der mindestens eine zusätzliche Schutzfilmbildner (I) ausgewählt aus der Gruppe bestehend aus teilverseiften Polyvinylalkoholen, Natriumcarboxymethylcellulose, Calciumcarboxymethylcellulose, Natriumpolyacrylat, Polyvinylpyrrolidon, Chitosan, Carboxymethylchitosan, Chitosanlactat, Polyester-16, Adipinsäure/Diethylentrimain Copolymer, Polyurethan-17 und Natriumpolystyrolsulfonat.

Teilverseifter Polyvinylalkohol ist auch bekannt unter dem INCI-Namen POLYVINYL ALCOHOL. Er trägt die CAS-Nummer 25213-24-5.

Natriumcarboxymethylcellulose ist auch bekannt unter dem INCI-Namen CELLULOSE GUM. Sie trägt die CAS-Nummer 9004-32-4.

Calciumcarboxymethylcellulose ist auch bekannt unter dem INCI-Namen calcium CALCIUM CARBOXYMETHYLCELLULOSE. Sie trägt die CAS-Nummer 9050-04-8.

Natriumpolyacrylat ist auch bekannt unter dem INCI-Namen SODIUM POLYACRYLATE. Es trägt die CAS-Nummern 9003-04-7 / 25549-84-2.

Polyvinylpyrrolidon ist auch bekannt unter dem INCI-Namen PVP. Es trägt die CAS-Nummer 9003-39-8.

Chitosan ist auch bekannt unter dem INCI-Namen CHITOSAN. Es trägt die CAS-Nummer 9012-76-4.

Carboxymethylchitosan ist auch bekannt unter dem INCI-Namen CARBOXYMETHYL CHITOSAN. Es trägt die CAS-Nummer 83512-85-0.

Chitosanlactat ist auch bekannt unter dem INCI-Namen CHITOSAN LACTATE. Es trägt die CAS-Nummer 66267-50-6.

Polyester-16 ist auch bekannt unter dem INCI-Namen POLYESTER-16. Es trägt die CAS-Nummer 69847-57-0.

Adipinsäure/Diethylentriamin Copolymer ist auch bekannt unter dem INCI-Namen ADIPIC ACID/DIETHYLENENETRIAMINE COPOLYMER. Es trägt die CAS-Nummer 25085-20-5.

Polyurethan-17 ist auch bekannt unter dem INCI-Namen POLYURETHANE-17. Es trägt die CAS-Nummer 347175-78-4.

Natriumpolystyrolsulfonat ist auch bekannt unter dem INCI-Namen SODIUM POLYSTYRENE SULFONATE. Es trägt die CAS-Nummern 9080-79-9 / 62744-35-8.

Die erfindungsgemäße wässrige Lösung (wL) kann nach allen dem Fachmann bekannten Methoden hergestellt werden. Beispielsweise können sämtliche in der wässrigen Lösung (wL) enthaltenen Komponenten (A) bis (E) sowie gegebenenfalls (F) bis (I) zueinander gegeben werden und anschließend vermischt werden. Diese Herstellung kann bei beliebiger Temperatur erfolgen. Bevorzugt erfolgt die Herstellung bei einer Temperatur im Bereich von 20 bis 95 °C, wobei die Temperatur während dem Zueinandergeben der Komponenten und/oder dem Mischen gegebenenfalls variiert werden kann.

Bevorzugt werden die Komponenten (A) bis (E) sowie gegebenenfalls (F) bis (I) nacheinander zugegeben und miteinander vermischt, gegebenenfalls bei erhöhter Temperatur. Dabei ist es insbesondere bevorzugt, dass zumindest bei der Zugabe der Komponente (C) die Temperatur im Bereich von 75 bis 90 °C liegt.

Insbesondere bevorzugt wird die wässrige Lösung (wL) nach einem Verfahren umfassend die folgenden Schritte a) bis d) hergestellt:
a) Bereitstellen einer ersten Mischung (M1), die einen ersten Teil der Komponente (A), die Komponente (C) und die Komponente (D) enthält bei einer Temperatur im Bereich von 75 bis 90 °C,
b) Zugabe eines zweiten Teils der Komponente (A) und der Komponente (B) zu der in Schritt a) hergestellten ersten Mischung (M1),
c) Mischen der in Schritt a) bereitgestellten ersten Mischung (M1) mit dem in Schritt b) zugegebenen zweiten Teil der Komponente (A) und der Komponente (B) bei einer Temperatur im Bereich von 20 bis 80 °C unter Erhalt eines Gemischs und
d) Zugabe der Komponente (E) zu dem Gemisch bei einer Temperatur im Bereich von 20 bis 40 °C unter Erhalt der wässrigen Lösung (wL).

Enthält die wässrige Lösung (wL) zusätzlich zumindest eine der Komponenten (F) bis (I), so können diese Komponenten ebenfalls zu einem beliebigen Zeitpunkt zugegeben werden.

Vorzugsweise umfasst ein Verfahren zur Herstellung der wässrigen Lösung (wL), die zusätzlich zumindest eine der Komponenten (F) bis (I) enthält, die folgenden Schritte i) bis x):
i) Bereitstellen einer ersten Mischung (M1), die einen ersten Teil der Komponente (A), die Komponente (C) und die Komponente (D) enthält bei einer Temperatur im Bereich von 75 bis 90 °C,
ii) Zugabe eines zweiten Teils der Komponente (A) und der Komponente (I) zu der in Schritt i) bereitgestellten ersten Mischung (M1),
iii) Mischen der in Schritt i) bereitgestellten ersten Mischung (M1) mit dem zweiten Teil der Komponente (A) und der Komponente (I) bei einer Temperatur im Bereich von 40 bis 90 °C unter Erhalt einer zweiten Mischung (M2),
iv) Zugabe eines dritten Teils der Komponente (A), der Komponente (B) und eines ersten Teils der Komponente (F) zu der in Schritt iii) erhaltenen zweiten Mischung (M2),
v) Mischen der in Schritt iii) erhaltenen zweiten Mischung (M2) mit dem dritten Teil der Komponente (A), der Komponente (B) und dem ersten Teil der Komponente (F) bei einer Temperatur im Bereich von 20 bis 70 °C unter Erhalt einer dritten Mischung (M3),
vi) Zugabe der Komponente (H) und eines zweiten Teils der Komponente (F) zu der in Schritt v) erhaltenen dritten Mischung (M3),
vii) Mischen der in Schritt v) erhaltenen dritten Mischung (M3) mit der Komponente (H) und dem zweiten Teil der Komponente (F) bei einer Temperatur im Bereich von 20 bis 40 °C unter Erhalt einer vierten Mischung (M4),
viii) Zugabe der Komponente (G) und eines dritten Teils der Komponente (F) zu der vierten Mischung (M4),
ix) Mischen der in Schritt vii) erhaltenen vierten Mischung (M4) mit der Komponente (G) und dem dritten Teil der Komponente (F) bei einer Temperatur im Bereich von 20 bis 40 °C unter Erhalt einer fünften Mischung (M5) und
x) Zugabe der Komponente (E) zu der fünften Mischung (M5) und Mischen der Komponente (E) und der fünften Mischung (M5) bei einer Temperatur im Bereich von 20 bis 40 °C unter Erhalt der wässrigen Lösung (wL).

Als erster Teil der Komponente (A), der in der in Schritt i) bereitgestellten ersten Mischung (M1) bereitgestellt wird, werden beispielsweise 40 bis 55 % der in der wässrigen Lösung (wL) enthaltenen Komponente (A) eingesetzt.

Der zweite Teil der Komponente (A), der in Schritt ii) zu der ersten Mischung (M1) zugegeben wird, sind beispielsweise 40 bis 55 % der in der wässrigen Lösung (wL) enthaltenen Komponente (A).

Der dritte Teil der Komponente (A), der in Schritt iv) zugegeben wird, sind beispielsweise 1 bis 20 % der in der wässrigen Lösung (wL) enthaltenen Komponente (A).

Es versteht sich von selbst, dass, wenn die wässrige Lösung (wL) zumindest eine der optionalen Komponenten (F) bis (I) nicht enthält, diese Komponente in dem vorstehend beschriebenen Verfahren umfassend die Schritte i) bis x) in dem entsprechenden Verfahrensschritt nicht zugegeben wird.

Gegenstand der vorliegenden Erfindung ist weiterhin ein wässriger Schaum (wS), der die erfindungsgemäße wässrige Lösung (wL) und mindestens ein Gas enthält.

"Mindestens ein Gas" bedeutet im Rahmen der vorliegenden Erfindung sowohl genau ein Gas als auch eine Mischung aus zwei oder mehreren Gasen.

Als das mindestens eine Gas eignen sich alle dem Fachmann bekannten Gase, die sich gegenüber den in der wässrigen Lösung (wL) enthaltenen Komponenten (A) bis (E) sowie gegebenenfalls (F) bis (I) inert verhalten. Bevorzugt ist das mindestens eine Gas kein Treibmittel.

Unter "Treibmittel" werden im Rahmen der vorliegenden Erfindung Kohlenwasserstoffe, insbesondere Fluorchlorkohlenwasserstoffe, Ether und Alkane verstanden, die bei einer Anwendungstemperatur der wässrigen Lösung (wL) und/oder des wässrigen Schaums (wS), also beispielsweise bei Temperaturen im Bereich von 15 bis 40 °C, bevorzugt im Bereich von 20 bis 30 °C, und bei Raumdruck, insbesondere bei einem Druck im Bereich von 900 bis 1200 mbar, gasförmig vorliegen und mit den in der wässrigen Lösung (wL) bzw. in dem wässrigen Schaum (wS) enthaltenen Komponenten (A) bis (F) keine chemische Reaktion eingehen, sich gegenüber den Komponenten (A) bis (I) also inert verhalten.

Besonders bevorzugt ist das mindestens eine Gas Luft.

Unter "Luft" wird im Rahmen der vorliegenden Erfindung das übliche Gasgemisch der Erdatmosphäre verstanden.

Der wässrige Schaum (wS) enthält bevorzugt im Bereich von 5 bis 40 Vol.-% der wässrigen Lösung (wL) und im Bereich von 60 bis 95 Vol.-% des mindestens einen Gases, jeweils bezogen auf das Gesamtvolumen des wässrigen Schaums (wS).

Es versteht sich von selbst, dass der wässrige Schaum (wS) die in der wässrigen Lösung (wL) enthaltenen Komponenten (A) bis (E) sowie gegebenenfalls (F) bis (I) ebenfalls enthält und dass für diese die vorstehenden Ausführungen und Bevorzugungen ebenfalls gelten.

Der wässrige Schaum (wS) kann nach allen dem Fachmann bekannten Methoden hergestellt werden. Vorzugsweise wird der wässrige Schaum (wS) hergestellt nach einem Verfahren umfassend die Schritte a) bis b):
a) Bereitstellen der erfindungsgemäßen wässrigen Lösung (wL),
b) Mischen der in Schritt a) bereitgestellten wässrigen Lösung (wL) mit dem mindestens einen Gas unter Erhalt des wässrigen Schaums (wS).

Das Mischen der wässrigen Lösung (wL) mit dem mindestens einen Gas erfolgt vorzugsweise bei Temperaturen, bei denen der erhaltene wässrige Schaum (wS) angewandt wird. Beispielsweise erfolgt das Mischen der wässrigen Lösung (wL) mit dem mindestens einen Gas bei einer Temperatur im Bereich von 0 bis 50 °C, bevorzugt im Bereich von 15 bis 30 °C.

Das Mischen der wässrigen Lösung (wL) mit dem mindestens einen Gas erfolgt, vorzugsweise indem mechanisch, beispielsweise in einer Spendervorrichtung, Überdruck erzeugt wird, beispielsweise ein Überdruck im Bereich von 0,05 bis 2 bar, wobei sich das mindestens eine Gas mit der wässrigen Lösung (wL) vermischt unter Erhalt des wässrigen Schaums (wS).

Gegenstand der vorliegenden Erfindung ist weiterhin eine Vorrichtung, bevorzugt eine Spendervorrichtung, umfassend die erfindungsgemäße wässrige Lösung (wL) und mindestens ein Gas.

Für das mindestens eine Gas gelten die zuvor beschriebenen Ausführungen und Bevorzugungen.

Als derartige Vorrichtungen, bevorzugt Spendervorrichtungen, eignen sich alle dem Fachmann bekannten Vorrichtungen, bevorzugt Spendervorrichtungen. Insbesondere bevorzugt sind Vorrichtungen, bevorzugt Spendervorrichtungen, die die wässrige Lösung (wL) innerhalb eines in die Spendervorrichtung einzusetzenden Behälters oder innerhalb eines fest in die Spendervorrichtung eingebauten Vorratsbehälters enthalten und bei denen das mindestens eine Gas, bevorzugt Luft, aus der Umgebung um die Vorrichtung, bevorzugt die um Spendervorrichtung, in die Vorrichtung, bevorzugt in die Spendervorrichtung, aufgenommen wird.

Gegenstand der vorliegenden Erfindung ist weiterhin die Verwendung der erfindungsgemäßen wässrigen Lösung (wL) als Hautschutzpräparat, vorzugsweise als Hautschutzpräparat gegen wasserlösliche und/oder wasserunlösliche Arbeitsstoffe, besonders bevorzugt als Hautschutzpräparat gegen wasserlösliche und wasserunlösliche Arbeitsstoffe.

Außerdem Gegenstand der vorliegenden Erfindung ist die Verwendung des erfindungsgemäßen wässrigen Schaums (wS) als Hautschutzpräparat, vorzugsweise als Hautschutzpräparat gegen wasserlösliche und/oder wasserunlösliche Arbeitsstoffe, besonders bevorzugt als Hautschutzpräparat gegen wasserlösliche und wasserunlösliche Arbeitsstoffe.

Unter Arbeitsstoffen werden im Rahmen der vorliegenden Erfindung Stoffe verstanden, die in direkten Kontakt mit der Haut gelangen können. Die Arbeitsstoffe können wasserlöslich oder wasserunlöslich sein. Besondere Bedeutung haben dabei Arbeitsstoffe, die durch langanhaltenden und/oder wiederholten Kontakt zu chronischen Hautschädigungen führen können. Beispiele für wasserlösliche Arbeitsstoffe sind wasserlösliche Alkohole, wässrige Lösungen von Reinigungsmitteln, verdünnte Säuren oder wassergemischte Kühlschmierstoffe. Beispiele für wasserunlösliche Arbeitsstoffe sind Öle, Kohlenwasserstoffe oder nicht wassermischbare Kühlschmierstoffe.

Die erfindungsgemäße wässrige Lösung (wL) sowie der erfindungsgemäße wässrige Schaum (wS) sind bevorzugt frei von Treibmitteln. Dies bedeutet, dass die wässrige Lösung (wL) sowie der wässrige Schaum (wS) bevorzugt kein Treibmittel enthalten. "Kein Treibmittel" bedeutet im Rahmen der vorliegenden Erfindung höchstens 1 Vol.-%, bevorzugt höchstens 0,5 Vol.-% und insbesondere bevorzugt höchstens 0,1 Vol.-% eines Treibmittels, bezogen auf das Gesamtvolumen der wässrigen Lösung (wL) bzw. des wässrigen Schaums (wS).

Weiterhin ist es bevorzugt, dass die wässrige Lösung (wL) sowie der wässrige Schaum (wS) keine wasserunlöslichen, (feste oder flüssige) Komponenten enthalten, die unter Verwendung geeigneter oberflächenaktiver Substanzen (Tenside) in der wässrigen Lösung dispergiert sind.

Es ist weiterhin bevorzugt, dass die wässrige Lösung (wL) sowie der wässrige Schaum (wS) keine Emulsionsstabilisatoren und/oder Suspensionsstabilisatoren enthalten. Unter "Emulsionsstabilisatoren" und Suspensionsstabilisatoren werden im Rahmen der vorliegenden Erfindung Verbindungen verstanden, die die Aufrechterhaltung der homogenen Verteilung der dispersen Phase in einer Emulsion oder Suspension unterstützen. Bei Emulsionen ist die disperse Phase fest, bei Suspensionen ist die disperse Phase flüssig. Bei den Emulsionsstabilisatoren und/oder Suspensionsstabilisatoren handelt es sich bevorzugt um geeignete Tenside und/oder Polymere.

Es ist erfindungsgemäß außerdem bevorzugt, dass die wässrige Lösung (wL) sowie der wässrige Schaum (wS) keine Siliconverbindungen enthalten, dass die wässrige Lösung (wL) sowie der wässrige Schaum (wS) also siliconfrei sind. Unter "Siliconverbindungen" werden im Rahmen der vorliegenden Erfindung organische Siliciumverbindungen, bevorzugt aus den Vertretern der Gruppe der Silane, der Silanole und der Siloxane verstanden.

## Patentansprüche

1. Wässrige Lösung (wL), enthaltend die Komponenten (A) bis (E):
(A) Wasser,
(B) mindestens ein Tensid, das ausgewählt ist aus der Gruppe bestehend aus amphoteren Tensiden, anionischen Tensiden und nichtionischen Tensiden,
(C) mindestens ein Fluorpolymer,
(D) mindestens einen ersten pH-Regulator, der basisch ist, und
(E) mindestens einen zweiten pH-Regulator, der sauer ist,
wobei die wässrige Lösung (wL) im Bereich von 70 bis 98 Gew.-% der Komponente (A), im Bereich von 0,1 bis 10 Gew.-% der Komponente (B), im Bereich von 0,1 bis 10 Gew.-% der Komponente (C), im Bereich von 0,01 bis 5 Gew.-% der Komponente (D) und im Bereich von 0,01 bis 5 Gew.-% der Komponente (E) enthält, jeweils bezogen auf das Gesamtgewicht der wässrigen Lösung (wL).

2. Wässrige Lösung (wL) gemäß Anspruch 1, **dadurch gekennzeichnet, dass**
i) das mindestens eine Tensid (B) ausgewählt ist aus der Gruppe bestehend aus Cocamidopropylbetain, Dinatriumlaurylethersulfosuccinat, Kokosbetain, Natriumkokosfettsäureamphoacetat, Dinatriumlaurylsulfosuccinat, Natriumlaurylsulfat, Natriumlaurylethersulfat, Natriumkokosfettsäuresulfat, MIPA-Laurylethersulfat, MEA-Laurylsulfat, Natriumlauroylglutamat, Dinatriumcocoylglutamat, Natriumlaurylsulfoacetat, Kokosglucosid, Laurylglucosid, Decylglucosid, Caprylylglukosid, Caprylyl/Capryl-Glucosid, Kokosfettsäurediethanolamid, Sojafettsäurediethanolamid und/oder
ii) das mindestens eine Fluorpolymer (C) ausgewählt ist aus Perfluorpolyethern, Polyurethan-26 und Polyurethan-27, vorzugsweise aus Perfluorpolyetherphosphaten, insbesondere bevorzugt aus Perfluorethoxymethoxy difluorethyl polyethylenglycol phosphaten.

3. Wässrige Lösung (wL) gemäß Anspruch 1 oder 2, **dadurch gekennzeichnet, dass**
i) der mindestens eine erste pH-Regulator (D) ausgewählt ist aus der Gruppe bestehend aus Natriumhydroxid, Kaliumhydroxid, Natriumcarbonat, Kaliumcarbonat, Trinatriumorthophosphat, Natriumtriphosphat und Kaliumtriphosphat und/oder
ii) der mindestens eine zweite pH-Regulator (E) ausgewählt ist aus der Gruppe bestehend aus Citronensäure, Milchsäure, Essigsäure, Ascorbinsäure, Phosphorsäure, Adipinsäure, Bernsteinsäure und Salzsäure.

4. Wässrige Lösung (wL) gemäß einem der Ansprüche 1 bis 3, **dadurch gekennzeichnet, dass** die wässrige Lösung (wL) zusätzlich zumindest eine der Komponenten
(F) mindestens einen wasserlöslichen Pflegestoff ausgewählt aus der Gruppe bestehend aus Glycerin, Betain, Caprylylglykol, Dexpanthenol, Propylenglykol, Propandiol, Butylglykol, Hyaluronsäure, Natriumhyaluronat, Allantoin, Decylenglykol, Harnstoff, L-Arginin, Honig, Ascorbinsäure, Nikotinsäure, Nikotinsäureamid, Milchprotein, Collagen und wasserlösliche, pflanzliche Extrakte, und/oder
(G) mindestens einen Duftstoff, und/oder
(H) mindestens einen Konservierungsstoff ausgewählt aus der Gruppe bestehend aus Phenoxyethanol, Benzylalkohol, Methylisothiazolinon, Chlormethylisothiazolinon, Dehydracetsäure, Natriumdehydracetat, Benzoesäure, Natriumbenzoat, Sorbinsäure, Kaliumsorbat, p-Hydroxybenzoesäuremethylester, p-Hydroxybenzoesäureethylester, p-Hydroxybenzoesäurepropylester, p-Hydroxybenzoesäurebutylester, Zinkpyrithion, Benzalkoniumchlorid, Bromnitropropandiol und lodpropinylbutylcarbamat, und/oder
(I) mindestens einen zusätzlichen Schutzfilmbildner ausgewählt aus der Gruppe bestehend aus teilverseiften Polyvinylalkoholen, Natriumcarboxymethylcellulose, Calciumcarboxymethylcellulose, Natriumpolyacrylat, Polyvinylpyrrolidon, Chitosan, Carboxymethylchitosan, Chitosanlactat, Polyester-16, Adipinsäure/Diethylentrimain Copolymer, Polyurethan-17 und Natriumpolystyrolsulfonat
enthält.

5. Wässrige Lösung (wL) gemäß einem der Ansprüche 1 bis 4, **dadurch gekennzeichnet, dass** die wässrige Lösung (wL) zusätzlich im Bereich von 0,1 bis 10 Gew.-% der Komponente (F) und/oder im Bereich von 0,02 bis 2 Gew.-% der Komponente (G) und/oder im Bereich von 0,001 bis 2 Gew.-% der Komponente (H) und/oder im Bereich von 0,1 bis 5 Gew.-% der Komponente (I) enthält, jeweils bezogen auf das Gesamtgewicht der wässrigen Lösung (wL).

6. Wässrige Lösung (wL) gemäß einem der Ansprüche 1 bis 5, **dadurch gekennzeichnet, dass** das Gewichtsverhältnis der Komponente (A) zur Komponente (B) im Bereich von 10 zu 1 bis 200 zu 1 liegt, bevorzugt im Bereich von 50 zu 1 bis 100 zu 1, insbesondere bevorzugt im Bereich von 60 zu 1 bis 80 zu 1.

7. Wässriger Schaum (wS), der die wässrige Lösung (wL) gemäß einem der Ansprüche 1 bis 6 und mindestens ein Gas enthält.

8. Wässriger Schaum (wS) gemäß Anspruch 7, **dadurch gekennzeichnet, dass** das mindestens eine Gas Luft ist.

9. Wässriger Schaum (wS) gemäß Anspruch 7 oder 8, **dadurch gekennzeichnet, dass** der wässrige Schaum (wS) im Bereich von 5 bis 20 Vol.-% der wässrigen Lösung (wL) und im Bereich von 80 bis 95 Vol.-% des mindestens einen Gases enthält, jeweils bezogen auf das Gesamtvolumen des wässrigen Schaums (wS).

10. Verfahren zur Herstellung einer wässrigen Lösung (wL) gemäß einem der Ansprüche 1 bis 6 umfassend die Schritte a) bis d)
a) Bereitstellen einer ersten Mischung (M1), die einen ersten Teil der Komponente (A), die Komponente (C) und die Komponente (D) enthält bei einer Temperatur im Bereich von 75 bis 90 °C,
b) Zugabe eines zweiten Teils der Komponente (A) und der Komponente (B) zu der ersten Mischung (M1),
c) Mischen der in Schritt a) bereitgestellten ersten Mischung (M1) mit dem in Schritt b) zugegebenen zweiten Teil der Komponente (A) und der Komponente (B) bei einer Temperatur im Bereich von 20 bis 80 °C unter Erhalt eines Gemischs,
d) Zugabe der Komponente (E) zu dem Gemisch bei einer Temperatur im Bereich von 20 bis 40 °C unter Erhalt der wässrigen Lösung (wL).

11. Vorrichtung, bevorzugt eine Spendervorrichtung, umfassend eine wässrige Lösung (wL) gemäß einem der Ansprüche 1 bis 6 und mindestens ein Gas, vorzugsweise Luft.

12. Verfahren zur Herstellung eines wässrigen Schaums (wS) gemäß einem der Ansprüche 7 bis 9 umfassend die Schritte
a) Bereitstellen einer wässrigen Lösung (wL) gemäß einem der Ansprüche 1 bis 6,
b) Mischen der in Schritt a) bereitgestellten wässrigen Lösung (wL) mit dem mindestens einen Gas unter Erhalt des wässrigen Schaums (wS).

13. Verwendung einer wässrigen Lösung (wL) gemäß einem der Ansprüche 1 bis 6 als Hautschutzpräparat, vorzugsweise als Hautschutzpräparat gegen wasserlösliche und/oder wasserunlösliche Arbeitsstoffe.

14. Verwendung eines wässrigen Schaums (wS) gemäß einem der Ansprüche 7 bis 9 als Hautschutzpräparat, vorzugsweise als Hautschutzpräparat gegen wasserlösliche und/oder wasserunlösliche Arbeitsstoffe.

## Claims

1. Aqueous solution (wL), containing components (A) to (E):
(A) water,
(B) at least one surfactant selected from the group consisting of amphoteric surfactants, anionic surfactants and nonionic surfactants,
(C) at least one fluoropolymer,
(D) at least one first, basic pH adjusting agent, and
(E) at least one second, acidic pH adjusting agent,
wherein the aqueous solution (wL) contains in the range from 70 to 98% by weight of component (A), in the range from 0.1 to 10% by weight of component (B), in the range from 0.1 to 10% by weight of component (C), in the range from 0.01 to 5% by weight of component (D) and in the range of 0.01 to 5% by weight of component (E), based in each case on the total weight of the aqueous solution (wL).

2. Aqueous solution (wL) according to claim 1, wherein
i) the at least one surfactant (B) is selected from the group consisting of cocamidopropyl betaine, disodium laureth sulfosuccinate, cocamidopropyl betaine, sodium cocoamphoacetate, disodium lauryl sulfosuccinate, sodium lauryl sulfate, sodium laureth sulfate, sodium coco-sulfate, MIPA laureth sulfate, MEA lauryl sulfate, sodium lauroyl glutamate, disodium cocoyl glutamate, sodium lauryl sulfoacetate, coco-glucoside, lauryl glucoside, decyl glucoside, capryl glucoside, caprylyl/capryl glucoside, coconut diethanolamide, soya diethanolamide, and/or
ii) the at least one fluoropolymer (C) is selected from perfluoropolyethers, polyurethane-26 and polyurethane-27, preferably from perfluoropolyether phosphates, especially preferred from perfluoroethoxymethoxy difluoroethyl polyethyleneglycol phosphates.

3. Aqueous solution (wL) according to claim 1 or 2, wherein
i) the at least one first pH adjusting agent (D) is selected from the group consisting of sodium hydroxide, potassium hydroxide, sodium carbonate, potassium carbonate, trisodium orthophosphate, sodium triphosphate and potassium triphosphate and/or
ii) the at least one second pH adjusting agent (E) is selected from the group consisting of citric acid, lactic acid, acetic acid, ascorbic acid, phosphoric acid, adipic acid, succinic acid and hydrochloric acid.

4. Aqueous sollution (wL) according to any of claims 1 to 3, wherein the aqueous solution (wL) contains additionally at least one of the components
(F) at least one water-soluble care substance selected from the group consisting of glycerine, betaine, caprylyl glycol, dexpanthenol, propylene glycol, propanediol, butyl glycol, hyaluronic acid, sodium hyaluronate, allantoin, decylene glycol, urea, L-arginine, honey, ascorbinic acid, nicotinic acid, nicotinic acid amide, milk protein, collagen and water-soluble, vegetal extracts, and/or
(G) at least one fragrance, and/or
(H) at least one preservative selected from the group consisting of phenoxyethanol, benzyl alcohol, methylisothiazolinone, chloromethyl-isothiazolinone, dehydroacetic acid, sodium dehydroacetate, benzoic acid, sodium benzoate, sorbic acid, potassium sorbate, methyl p-hydroxybenzoate, ethyl p-hydroxybenzoate, propyl p-hydroxybenzoate, butyl p-hydroxybenzoate, zinc pyrithione, benzalkonium chloride, bromonitro-propanediol and iodopropynylbutyl carbamate, and/or
(I) at least one additional protective film former selected from the group consisting of partially saponified polyvinyl alcohols, sodium carboxymethylcellulose, calcium carboxymethyl cellulose, sodium polyacrylate, polyvinyl pyrrolidone, chitosan, carboxymethyl chitosan, chitosan lactate, polyester-16, adipic acid/diethylene triamine copolymer, polyurethane-17 and sodium polystyrene sulfonate.

5. Aqueous solution (wL) according to any of claims 1 to 4, wherein the aqueous solution (wL) contains additionally in the range from 0.1 to 10% by weight of component (F) and/or in the range fro 0.02 to 2% by weight of component (G) and/or in the range of 0.001 to 2% by weight of component (H) and/or in the range from 0.1 to 5% by weight of component (I), based in each case on the total weight of the aqueous solution (wL).

6. Aqueous solution (wL) according to any of claims 1 to 5, wherein the weight ratio of component (A) to component (B) is in the range from 10 : 1 to 200 : 1, preferably in the range from 50 : 1 to 100 : 1, particularly preferred in the range from 60 : 1 to 80 : 1.

7. Aqueous foam (wS) containing the aqueous solution (wL) according to any of claims 1 to 6 and at least one gas.

8. Aqueous foam (wS) according to claim 7, wherein the at least one gas is air.

9. Aqueous foam (wS) according to claim 7 or 8, **characterized in that** the aqueous foam (wS) contains in the range from 5 to 20% by volume of the aqueous solution (wL) and in the range from 80 to 95% by volume of the at least one gas, based in each case on the total volume of the aqueous foam (wS).

10. Method for producing an aqueous solution (wL) according to any of claims 1 to 6, comprising the steps a) to d)
a) providing a first mixture (M1) containing a first part of component (A), component (C) and component (D) at a temperature in the range from 75 to 90°C,
b) adding a second part of component (A) and component (B) to the first mixture (M1),
c) mixing the first mixture (M1) provided in step a) with the second part of component (A) and component (B) provided in step b) at a temperature in the range from 20 to 80°C, thereby obtaining a further mixture,
d) adding component (E) to the mixture obtained in step c) at a temperature in the range from 20 to 40°C, thereby obtaining the aqueous solution (wL).

11. Apparatus, preferably a dispensing apparatus, comprising an aqueous solution (wL) according to any of claims 1 to 6, and at least one gas, preferably air.

12. Method for producing an aqueous foam (wS) according to any of claims 7 to 9, comprising the steps
a) providing an aqueous solution (wL) according to any of claims 1 to 6,
b) mixing the aqueous solution (wL) provided in step a) with the at least one gas, thereby obtaining the aqueous foam (wS).

13. Use of an aqueous solution (wL) according to any of claims 1 to 6 as a skin protection product, preferably as a skin protection product against water-soluble and/or water-insoluble working materials.

14. Use of an aqueous foam (wS) according to any of claims 7 to 9 as a skin protection product, preferably as a skin protection product against water-soluble and/or water-insoluble working materials.

## Revendications

1. Solution aqueuse (wL) contenant les composants (A) à (E) :
(A) de l'eau
(B) au moins un tensioactif choisi dans le groupe constitué par les tensioactifs amphotères, les tensioactifs anioniques et les tensioactifs non ioniques,
(C) au moins un polymère fluoré,
(D) au moins un premier régulateur de pH qui est basique, et
(E) au moins un deuxième régulateur de pH qui est acide,
dans laquelle la solution aqueuse (wL) comprend dans la plage de 70 à 98% en poids du composant (A), dans la plage de 0,1 à 10% en poids du composant (B), dans la plage de 0,1 à 10% en poids du composant (C), dans la plage de 0,01 à 5% en poids du composant (D) et dans la plage de 0,01 à 5% en poids du composant (E), chaque fois au rapport du poids total de la solution aqueuse (wL).

2. Solution aqueuse (wL) selon la revendication 1, dans laquelle
i) le ou les tensioactifs (B) sont choisis dans le groupe constitué par le bétaïne de cocamidopropyle, le laureth sulfosuccinate disodique, le coco-bétaïne, le cocoamphoacétate de sodium, le lauryl sulfosuccinate disodique, le laurylsulfate de sodium, le laureth sulfate de sodium, le sodium coco sulfate, le MIPA-laureth sulfate, le MEA-lauryl sulfate, le sodium lauroyl glutamate, le disodium cocoyl glutamate, le sodium lauryl sulfoacétate, le coco-glucoside, le lauryl glucoside, le décyl glucoside, le caprylyl glucoside, le caprylyl/capryl glucoside, le diéthanolamide d'acide gras de coco, le diéthanolamide d'acide gras de soja, et/ou
ii) le ou les polymères fluorés (C) sont choisis parmi les polyéthers perfluorés, le polyuréthane-26 et le polyuréthane-27, préférablement parmi les phosphates de perfluoropolyéthers, particulièrement préférablement parmi les phosphates de perfluoroéthoxyméthoxy difluoroéthyl polyéthylène glycol.

3. Solution aqueuse (wL) selon la revendication 1 ou 2, dans laquelle
i) le ou les premiers régulateurs de pH (D) sont choisis dans le groupe constitué par l'hydroxyde de sodium, l'hydroxyde de potassium, le carbonate de sodium, le carbonate de potassium, l'orthophosphate trisodique, le triphosphate de sodium et le triphosphate de potassium et/ou
ii) le ou les deuxièmes régulateurs de pH (E) sont choisis dans le groupe constitué par l'acide citrique, l'acide lactique, l'acide acétique, l'acide ascorbique, l'acide phosphorique, l'acide adipique, l'acide succinique et l'acide hydrochloride.

4. Solution aqueuse (wL) selon l'une quelconque des revendications 1 à 3, dans laquelle la solution aqueuse (wL) contient en outre au moins un des composants
(F) au moins un principe actif traitant soluble dans l'eau, choisi dans le groupe constitué par le glycérine, le bétaïne, le glycol caprylique, le dexpanthénol, le propylène glycol, le propanediol, le butylglycol, l'acide hyaluronique, l'hyaluronate de sodium, l'allantoïne, le décylène glycol, l'urée, le L-arginine, le miel, l'acide ascorbique, l'acide nicotinique, l'amide d'acide nicotinique, le protéine du lait, le collagène et les extraits végétaux, solubles dans l'eau, et/ou
(G) au moins une fragrance, et/ou
(H) au moins un conservateur choisi dans le groupe constitué par le phénoxyéthanol, l'alcool benzylique, le méthylisothiazolinone, le chlorométhylisothiazolinone, l'acide déhydroacétique, le déhydroacétate de sodium, l'acide benzoïque, le benzoate de sodium, l'acide sorbique, le sorbate de potassium, le p-hydroxybenzoate de méthyle, le p-hydroxybenzoate d'éthyle, le p-hydroxybenzoate de propyle, le p-hydroxybenzoate de butyle, le pyrithione de zinc, le chlorure de benzalkonium, le bromonitropropanediol et le iodopropynyle butyle carbamate, et/ou
(I) au moins un agent engendrant un film protecteur choisi par le groupe constitué par les alcools polyvinyliques partiellement saponifiés, la carboxyméthylcellulose sodique, la carboxyméthylcellulose de calcium, le polyacrylate de sodium, le polyvinylpyrrolidone, le chitosane, le carboxyméthyl chitosane, l'acétate de chitosane, le polyester-16, le copolymer d'acide adipique/diéthylènetriamine, le polyuréthane-17 et le polystyrène sulfonate de sodium.

5. Solution aqueuse (wL) selon l'une quelconque des revendications 1 à 4, dans laquelle la solution aqueuse (wL) contient en outre dans la plage de 0,1 à 10% en poids du composant (F) et/ou dans la plage de 0,02 à 2 % en poids du composant (G) et/ou dans la plage de 0,001 à 2% en poids du composant (H) et/ou dans la plage de 0,1 à 5% en poids du composant (I), chaque fois par rapport au poids total de la solution aqueuse (wL).

6. Solution aqueuse (wL) selon l'une quelconque des revendications 1 à 5 dans laquelle le rapport pondéral du composant (A) au composant (B) est dans la plage de 10 : 1 à 200 : 1, préférablement dans la plage de 50 : 1 à 100 : 1, particulièrement préférablement dans la plage de 60 : 1 à 80 : 1.

7. Mousse aqueuse (wS) contenant la solution aqueuse (wL) selon l'une quelconque des revendiations 1 à 6 et au moins un gaz.

8. Mousse aqueuse (wS) selon la revendication 7 dans laquelle le ou les gaz sont de l'air.

9. Mousse aqueuse (wS) selon la revendication 7 ou 8 dans laquelle la mousse aqueuse (wS) contient dans la plage de 5 à 20% en volume de la solution aqueuse (wL) et dans la plage de 80 à 95% en volume du ou des gaz, chaque fois par rapport au volume total de la mousse aqueuse (wS).

10. Méthode pour la production d'une solution aqueuse (wL) selon l'une quelconque des revendiations 1 à 6 comprenant les étapes a) à d)
a) fournir un premier mélange (M1) comprenant un premier part du composant (A), le composant (C) et le composant (D) à une température dans la plage de 75 à 90°C,
b) ajouter un deuxième part du composant (A) et du composant (B) au premier mélange (M1),
c) mélanger le mélange (M1) fourni à l'étape a) avec le deuxième part du composant (A) ajouté à l'étape b) et le composant (B) à une température dans la plage de 20 à 80°C pour obtenir un nouveau mélange,
d) ajouter le composant (E) au mélange obtenu à l'étape c) à une température dans la plage de 20 à 40°C pour obtenir la solution aqueuse (wL).

11. Dispositif, préférablement un dispositif de distribution, comprenant une solution aqueuse (wL) selon l'une quelconque des revendications 1 à 6 et au moins un gaz, préférablement de l'air.

12. Méthode pour la production d'une mousse aqueuse (wS) selon l'une quelconque des revendications 7 à 9, comprenant les étapes
a) fournir une solution aqueuse (wL) selon l'une quelconque des revendications 1 à 6,
b) mélanger la solution aqueuse (wL) fournie à l'étape a) avec le ou les gaz pour obtenir la mousse aqueuse (wS).

13. Utilisation d'une solution aqueuse (wL) selon l'une quelconque des revendications 1 à 6 en tant que dermo-protecteur, préférablement en tant que dermo-protecteur contre les agents solubles et/ou insolubles dans l'eau.

14. Utilisation d'une mousse aqueuse (wS) selon l'une quelconque des revendications 7 à 9 en tant que dermo-protecteur, préférablement en tant que dermo-protecteur contre les agents solubles et/ou insolubles dans l'eau.
